# EUROPÄISCHE PATENTANMELDUNG

(11) **EP 4 549 940 A1**
(43) Veröffentlichungstag der Anmeldung: **07.05.2025**
(21) Anmeldenummer: 24208831.8
(22) Anmeldetag: 25.10.2024
(51) Int. Cl.: G01N 33/543

(54) **VERFAHREN ZUR BESTIMMUNG DER IMMUNOGENITÄT EINES ANTIGENS**

(30) Priorität: 01.11.2023 DE 102023130217
(71) Anmelder: Universität Leipzig, Körperschaft des Öffentlichen Rechts, 04109 Leipzig (DE)
(72) Erfinder: Harzer, Maxi, 04109 Leipzig (DE); Vahlenkamp, Thomas, 04109 Leipzig (DE)
(74) Vertreter: Riechelmann & Carlsohn Patentanwälte PartG mbB

(57) **Zusammenfassung**

Die Erfindung betrifft ein Verfahren zur In-vitro-Bestimmung der Immunogenität eines ersten Antigens, das als Testantigen bezeichnet wird, wobei das Verfahren umfasst:
(a) Selektieren zumindest eines ersten Moleküls durch Inkubieren des Testantigens mit einem Molekülgemisch, das das zumindest eine erste Molekül und zumindest ein zweites Molekül enthält, um die Adsorption des zumindest einen ersten Moleküls oder des zumindest einen zweiten Moleküls an dem Testantigen zu ermöglichen,
(b) Durchführen eines Neutralisationstests unter Verwendung eines zweiten Antigens, das als Zielantigen bezeichnet wird, und unter Verwendung des in Schritt (a) selektierten, zumindest einen ersten Moleküls unter Erhalt eines Neutralisationstiters; und
(c) Bestimmen der Immunogenität des Testantigens unter Verwendung des in Schritt (b) erhaltenen Neutralisationstiters.

## Beschreibung

Die Erfindung betrifft ein Verfahren zur Bestimmung der Immunogenität eines Antigens. Sie betrifft ferner ein Kit, das zur Bestimmung der Immunogenität eines Antigens eingesetzt werden kann.

Unter dem Begriff "Immunogenität eines Antigens" versteht man die Fähigkeit des Antigens, bei Applikation in einen Organismus eine schützende Immunantwort auslösen zu können. Die schützende Immunantwort kann sich z. B. durch die Bildung neutralisierender Antikörper zeigen, die Epitop-spezifisch sind. Epitope sind molekulare Strukturen, beispielsweise Molekülabschnitte, eines Antigens, die eine spezifische Immunantwort - die Bildung spezifischer, neutralisierender Antikörper - auslösen können. Nach dem Stand der Technik wird sich mittels unterschiedlicher Methoden der Analyse immunogener Eigenschaften von Antigenen, insbesondere der verschiedenen Epitope, angenähert. Zu diesen Methoden gehören die als "Epitope Mapping" bezeichnete Bestimmung von Epitopen auf einem Antigen. Das kann beispielsweise durch das von dem Unternehmen PepScan entwickelte Verfahren erfolgen, bei dem eine als CLISP bezeichnete Epitopkartierungsplattform eingesetzt werden kann. Zu diesen Methoden gehören ferner die Röntgen-Kristallstrukturanalyse, die Kernspinresonanzspektroskopie, der Label-Transfer, der Protection Assay und der Alanin-Scan. Unter dem Begriff "Protection Assay" wird ein Verfahren zur Bestimmung von Molekülstrukturen, die für Enzyme unzugänglich sind, verstanden.

Die bekannten Methoden können unterteilt werden in solche, die lediglich lineare Epitope erkennen, und solche, die sowohl lineare als auch nicht-lineare (d. h. diskontinuierliche) Epitope erkennen. Allen bekannten Methoden gemeinsam ist, dass nur die Bindungsfähigkeit von Antikörpern beurteilt wird, aber keine Aussage darüber möglich ist, ob das Epitop zur Induktion von schützenden, d. h. neutralisierenden Antikörpern führt.

Darüber hinaus ist die Testung auf mögliche Immunogenität eines Antigens zur Stimulation einer schützenden Immunantwort bisher nur unter Verwendung von Versuchstieren möglich. Die Verwendung von Versuchstieren ist einerseits aufwändig und soll andererseits aufgrund ethischer Erwägungen und europäisch geltender Richtlinien nur dann erfolgen, wenn keine Alternativen zugänglich sind.

Aufgabe der Erfindung ist es, die Nachteile nach dem Stand der Technik zu beseitigen. Es soll insbesondere ein Verfahren zur Bestimmung der Immunogenität eines Antigens angegeben werden, das in vitro durchgeführt werden kann.

Diese Aufgabe wird durch die Merkmale der Ansprüche 1 und 12 gelöst. Zweckmäßige Ausgestaltungen der Erfindungen ergeben sich aus den Merkmalen der Unteransprüche.

Nach Maßgabe der Erfindung ist ein Verfahren zur In-vitro-Bestimmung der Immunogenität eines ersten Antigens, das als Testantigen bezeichnet wird, vorgesehen, wobei das Verfahren umfasst:
(a) Selektieren zumindest eines ersten Moleküls durch Inkubieren des Testantigens mit einem Molekülgemisch, das das zumindest eine erste Molekül und zumindest ein zweites Molekül enthält, um die Adsorption des zumindest einen ersten Moleküls oder des zumindest einen zweiten Moleküls an dem Testantigen zu ermöglichen,
(b) Durchführen eines Neutralisationstests unter Verwendung eines zweiten Antigens, das als Zielantigen bezeichnet wird, und unter Verwendung des in Schritt (a) selektierten, zumindest einen ersten Moleküls unter Erhalt eines Neutralisationstiters; und
(c) Bestimmen der Immunogenität des Testantigens unter Verwendung des in Schritt (b) erhaltenen Neutralisationstiters.

Das erfindungsgemäße Verfahren ermöglicht die In-Vitro-Bestimmung der Immunogenität eines Antigens, nämlich des als erstes Antigen bezeichneten Testantigens. Auf die Durchführung von Tierversuchen kann deshalb verzichtet werden. Durch das Vorschalten einer Adsorption des Molekülgemisches an ein Testantigen kann im nachfolgenden Neutralisationstest durch den Neutralisationstiter die immunologische Fähigkeit des Testantigens in-vitro beurteilt werden.

Das erfindungsgemäße Verfahren umfasst eine erste Ausführungsform und eine zweite Ausführungsform, die nachstehend gesondert erläutert werden. Dabei wird die erste Ausführungsform des erfindungsgemäßen Verfahrens als erstes erfindungsgemä-ßes Verfahren, die zweite Ausführungsform des erfindungsgemäßen Verfahrens als zweites erfindungsgemäßes Verfahren bezeichnet. Das erste erfindungsgemäße Verfahren sieht eine negative Selektion des ersten Moleküls vor, während das zweite erfindungsgemäße Verfahren eine positive Selektion des ersten Moleküls vorsieht.

Der Begriff "negative Selektion des ersten Moleküls" beschreibt den Umstand, dass es sich bei dem ersten Molekül, welches in Schritt (a) selektiert wird, um zumindest ein erstes Molekül handelt, das nicht an dem Testantigen adsorbiert wird. Adsorbiert an dem Testantigen wird zumindest ein zweites Molekül. Neben der Adsorption des zumindest einen zweiten Moleküls adsorbiert bevorzugt auch mindestens ein erstes Molekül an das Testantigen. Eine negative Selektion ist insbesondere für erste Moleküle zweckmäßig, die sich nach der Adsorption in Schritt (a) nicht vermehren lassen.

Der Begriff "positive Selektion des ersten Moleküls" beschreibt den Umstand, dass es sich bei dem ersten Molekül, das in Schritt (a) selektiert wird, um ein Molekül handelt, das an dem Testantigen adsorbiert wird. Es ist bevorzugt, dass nur erste Moleküle adsorbiert werden. Es kann zu diesem Zweck vorgesehen sein, dass das Testantigen so gewählt wird, dass an ihm bevorzugt nur das erste Molekül, aber nicht das zweite Molekül adsorbiert wird. Eine positive Selektion ist insbesondere für erste Moleküle zweckmäßig, die sich nach der Adsorption in Schritt (a) vermehren lassen, beispielsweise bei Verwendung von Phagen*-Displays.*

Das Molekülgemisch kann mehrere erste Moleküle enthalten. Es kann mehrere zweite Moleküle enthalten. Der Ausdruck "erstes Molekül" bezieht sich auf den Umstand, dass das erste Molekül in Schritt (a) selektiert wird, und zwar entweder durch negative Selektion oder durch positive Selektion. Der Ausdruck "zweites Molekül" bezieht sich auf den Umstand, dass das zweite Molekül in Schritt (a) nicht selektiert wird. Das Molekülgemisch kann beispielsweise Blut, Blutserum oder eine andere Körperflüssigkeit des menschlichen oder tierischen Körpers oder eine Präparation einer Phagen-*Display*-Bibliothek sein.

Das Molekülgemisch enthält das zumindest eine erste Molekül und ein zweites Molekül. Das Molekülgemisch kann jeweils mehr als ein erstes und/ oder mehr als ein zweites Molekül umfassen. Es kann vorgesehen sein, dass das oder die ersten Moleküle jeweils ein Protein sind. Es kann vorgesehen sein, dass das oder die zweiten Moleküle jeweils ein Protein sind. Vorzugsweise sind sowohl das oder die ersten Moleküle jeweils ein Protein als auch das oder die zweiten Moleküle jeweils ein Protein. Das oder die ersten Moleküle können jeweils ein Antikörper sein. Das oder die zweiten Moleküle können jeweils ein Antikörper sein. Vorzugsweise sind sowohl das oder die ersten Moleküle jeweils ein Antikörper als auch das oder die zweiten Moleküle jeweils ein Antikörper. Es ist bevorzugt, dass die ersten Moleküle Epitop-spezifische, neutralisierende Antikörper gegen das zu untersuchende Testantigen sind. Es ist weiterhin bevorzugt, dass die zweiten Moleküle für das Testantigen spezifische Antikörper sind, aber keine neutralisierenden Eigenschaften besitzen. Die ersten Moleküle unterscheiden sich chemisch von den zweiten Molekülen. Um chemisch unterschiedliche Moleküle handelt es sich im Sinne der Erfindung insbesondere bei Molekülen mit verschiedener Aminosäurereihung. Neben den ersten und zweiten Molekülen können weitere Moleküle, d. h. z. B. dritte, vierte oder fünfte Moleküle, die sich chemisch von dem ersten und zweiten Molekül und untereinander unterscheiden, im Molekülgemisch enthalten sein. Die weiteren Moleküle können ebenfalls für das Testantigen spezifisch sein, besitzen aber keine gegen das Epitop neutralisierende Wirkung.

Das erfindungsgemäße Verfahren sieht initial (d. h. in Schritt (a)) die Adsorption von bindungsfähigen Molekülen, z. B. Antikörpern, an ihr Antigen, das Testantigen, vor. Abhängig von der verwendeten Absorptionsmethodik, d. h. einer negativen oder positiven Selektion, werden nach der Adsorption am Testantigen die nicht gebundenen (Negativ-Selektion) oder die gebundenen (Positiv-Selektion) ersten Moleküle in nachfolgenden Schritten, also den Schritten (b) und (c), genutzt.

Nachstehend werden Einzelheiten des ersten erfindungsgemäßen Verfahrens und zweiten erfindungsgemäßen Verfahrens erläutert.

### Erstes erfindungsgemäßes Verfahren

Schritt (a) des ersten erfindungsgemäßen Verfahrens kann zumindest die Teilschritte umfassen:
(a1) Bereitstellen eines Serums, das das Molekülgemisch enthält; und
(a2) Inkubieren des in Schritt (a) bereitgestellten Serums mit dem Testantigen, um die Adsorption des zumindest einen zweiten Moleküls an dem Testantigen zu ermöglichen, zur Selektion des zumindest einen ersten Moleküls, das nicht an das Testantigen bindet.

Das in Schritt (a1) bereitgestellte Serum enthält das Molekülgemisch. Schritt (a1) sieht somit vor, dass das bereitgestellte Serum ein Serum ist, das zumindest ein erstes Molekül und zumindest ein zweites Molekül enthält. Das Serum selbst kann als Molekülgemisch angesehen werden. Dabei kann das Serum zusätzliche Komponenten enthalten. Das Serum kann ein antikörperspezifisches Serum sein.

Schritt (a2) beschreibt die negative Selektion des oder der ersten Moleküle, die nicht an das Testantigen binden. Zumindest ein erstes Molekül wird nicht an dem Testantigen adsorbiert, während zumindest ein zweites Molekül an dem Testantigen adsorbiert wird. In einer Ausführungsform adsorbieren einige der ersten Moleküle an das Testantigen, einige der ersten Moleküle nicht. In welchem Maß die ersten Moleküle adsorbiert werden, hängt von der Beschaffenheit des Testantigens ab. Es ist im Sinne der Erfindung bevorzugt, wenn möglichst viele der ersten Moleküle an das Testantigen binden, was für eine gute Immunogenität des Testantigens spricht.

Ziel von Schritt (b1) ist die Bindung der ersten Moleküle. Zur Bestimmung der Neutralisationseigenschaften der Testantigene wird nur eben jener Überstand an ersten Molekülen genutzt, die nicht gebunden werden. Auf diese Weise kann der Neutralisationstiter genutzt werden, um eine Aussage darüber zu treffen, wie gut das Testantigen neutralisierende erste Moleküle, beispielsweise neutralisierende Antikörper, binden kann. Wenn kein erstes Molekül binden kann, wären die Testantigene nicht von Nutzen und damit nicht wirkungsvoll.

Es ist theoretisch möglich, dass alle ersten Moleküle an das Testantigen binden, die Wahrscheinlichkeit dafür ist allerdings extrem gering ist. Zur Ausführung des ersten erfindungsgemäßen Verfahrens kann vorgesehen sein, dass zumindest eines der ersten Moleküle nicht an das Testantigen bindet. In Schritt (a2) muss mindestens ein zweites Molekül adsorbieren und mindestens ein erstes Molekül nicht.

Schritt (c) des ersten erfindungsgemäßen Verfahrens kann einen Vergleich des in Schritt (b) bestimmten Neutralisationstiters mit einem Vergleichs-Neutralisationstiter umfassen. Dabei kann der Vergleichsneutralisationstiter durch Durchführen eines Neutralisationstests unter Verwendung der in dem Serum enthaltenen Moleküle und unter Verwendung des Zielantigens bestimmt werden. Die zur Bestimmung des Vergleichsneutralisationstiters verwendeten Moleküle können somit das oder die ersten Moleküle und das oder die zweiten Moleküle umfassen.

Im Folgenden wird das erste erfindungsgemäße Verfahren mit der Maßgabe beschrieben, dass es sich bei dem oder den ersten Molekülen und bei dem oder den zweiten Molekülen jeweils um Antikörper handelt. Die Antikörper, die als erste Moleküle bezeichnet werden, sind neutralisierende Antikörper, die gegen ein bestimmtes Epitop des Testantigens binden. Die Antikörper, die als zweite Moleküle bezeichnet werden, sind gegen das Testantigen gerichtete spezifische Antikörper, die keine neutralisierende Wirkung besitzen. Das erste erfindungsgemäße Verfahren ist durch eine negative Selektion gekennzeichnet. Selektiert werden Epitop-spezifische, neutralisierende Antikörper, die nicht an das Testantigen binden. Antikörperspezifische Seren, die im oben angeführten Fall als Molekülgemisch bezeichnet werden, enthalten mehrere erste, zweite und weitere Moleküle, d. h. verschiedene Antikörper. An das Testantigen binden unter anderem je nach neutralisierender Eigenschaft des Epitops oder der Epitope des Testantigens eine bestimmte Anzahl der für diese/s Epitop/e-spezifischen neutralisierenden Antikörper. Die Anzahl richtet sich nach der immunologischen Eigenschaft des Testantigens. Je besser diese ist, umso mehr Epitop-spezifische, neutralisierende Antikörper werden gebunden. Die ungebundenen Epitop-spezifischen, neutralisierenden Antikörper, sind diejenigen, die für die folgende Untersuchung im Neutralisationstest von Interesse sind.

Die nicht vom Testantigen adsorbierten Antikörper werden nachfolgend in einem Zielantigen-spezifischen Neutralisationstest eingesetzt. Dadurch können Rückschlüsse auf die Immunogenität des Testantigens getroffen werden. Je mehr Epitop-spezifische, neutralisierende Antikörper in der Lage sind, an das Testantigen zu adsorbieren, d. h. umso weniger für den hier beschriebenen Schritt selektiert wurden, umso besser ist die immunologische Eignung des Testantigens, Epitop-spezifische, neutralisierende Antikörper zu induzieren.

Die Trennung der bindenden Antikörper von den nicht-bindenden Antikörpern kann durch ein geeignetes Adsorptionsverfahren, welches von der Präparation eines Testantigens abhängig ist, gewährleistet werden. Dabei kann das Testantigen in Lösung vorliegen oder immobilisiert an verschiedenen Oberflächen.

Eine Immobilisation eines Testantigens vor Inkubation mit Antikörpern kann dabei ermöglichen, dass eventuell schädliche Einflüsse der Testantigenpräparation auf den Neutralisationstest, z. B. Eigenschaften des Testantigens selber oder der ihm beigemischten Substanzen, verhindert werden. Eine Immobilisation des Testantigens zur Absorption von Antikörpern ist erfindungsgemäß bevorzugt zu wählen, wenn das Testantigen das Ergebnis des nachfolgenden Neutralisationstests beeinflussen würde. Durch die Adsorption von Epitop-spezifischen, neutralisierenden Antikörpern an das Testantigen wird die verfügbare Menge an neutralisierenden Antikörpern, die gegen das Zielantigen gerichtet sind, vermindert, so dass die in Lösung verbliebenen Antikörper in Schritt (b) nun einen geringeren Neutralisationstiter aufweisen. Testantigene, die keine neutralisierenden Antikörper adsorbieren, sind dagegen nicht dazu in der Lage, den Neutralisationstiter eines Antikörpers zu verringern.

Das erste erfindungsgemäße Verfahren bildet *in vitro* die Bestimmung der Immunogenität des Testantigens im Vergleich zum Zielantigen nach. Die Kombination der Adsorption von Antikörpern an ein zu evaluierendes Testantigen als vorgeschalteten Schritt vor der Durchführung eines Neutralisationstests ermöglicht erstmals die Beurteilung der immunologischen Fähigkeiten eines Testantigens in-vitro, sodass kostspielige und langwierige Tierversuche auf ein Mindestmaß reduziert werden können. Das erfindungsgemäße Verfahren erlaubt mithin die Beurteilung von Epitopen ex-vivo, insbesondere deren Fähigkeit, neutralisierende Antikörper zu induzieren.

### Zweites erfindungsgemäßes Verfahren

Schritt (a) des zweiten erfindungsgemäßen Verfahrens kann zumindest die Teilschritte umfassen:
(a1') Selektieren des zumindest einen ersten Moleküls, das an das Zielantigen bindet, durch Inkubieren des Molekülgemisches mit dem Zielantigen, um die Adsorption des zumindest einen ersten Moleküls an dem Zielantigen zu ermöglichen;
(a2') Vermehren des zumindest einen ersten Moleküls, das in Schritt (a1') an das Zielantigen binden konnte; und
(a3') Durchführen eines Neutralisationstests zur Bestimmung einer neutralisierenden Eigenschaft des in Schritt (a2') vermehrten, zumindest einen ersten Moleküls unter Erhalt eines Neutralisationstiters.

Dabei kann vorgesehen sein, dass in Schritt (a1') das Molekülgemisch als ein Serum bereitgestellt wird. Schritt (a1') beschreibt die positive Selektion des ersten Moleküls.

Das zweite erfindungsgemäße Verfahren kann im Anschluss an Schritt (a3') die Teilschritte umfassen:
(a4') Inkubieren des in Schritt (a1') selektierten, zumindest einen ersten Moleküls, wenn in Schritt (a3') ein Neutralisationstiter erhalten wird, mit dem Testantigen; und
(a5') Vermehren des zumindest einen ersten Moleküls, das in Schritt (a4') an das Testantigen binden konnte.
Schritt (a4') beschreibt die positive Selektion des ersten Moleküls.

Das zweite erfindungsgemäße Verfahren, also die positive Selektion, hat gegenüber dem ersten erfindungsgemäßen Verfahren, also der negativen Selektion, diverse Vorteile. Für das erste erfindungsgemäße Verfahren kann eine möglichst reine Antikörper-Präparation, d. h. eine solche, die neben den für das Testantigen spezifischen Antikörpern möglichst keine weiteren Antikörper enthält, vorgesehen sein. Eine solche möglichst reine Präparation ist im zweiten erfindungsgemäßen Verfahren nicht erforderlich, da mittels der positiven Selektion spezifisch diejenigen Antikörper für den Einsatz im Neutralisationstest selektiert werden, die an das Testantigen binden. Zu den Vorteilen gehört außerdem, dass ein Vergleich von Neutralisationstitern entfällt. Bei dem zweiten erfindungsgemäßen Verfahren beweist ein neutralisierender Titer der durch das Testantigen selektierten Antikörper die Immunogenität, unabhängig von der Höhe der Titer. Zu den Vorteilen des zweiten erfindungsgemäßen Verfahrens gehört ferner, dass die erforderlichen Techniken zu seiner Umsetzung bereits in größeren Firmen etabliert sind.

Im Folgenden wird das zweite erfindungsgemäße Verfahren mit der Maßgabe beschrieben, dass es sich bei dem oder den ersten Molekülen und bei dem oder den zweiten Molekülen jeweils um Antikörper handelt. Das zweite erfindungsgemäße Verfahren ist durch eine positive Selektion gekennzeichnet. Selektiert werden die Antikörper, die an das Testantigen binden. Die Antikörper, die an das Testantigen binden, werden nach der Adsorption vermehrt. Die positive Selektion erfolgt bevorzugt unter Verwendung der Phagen-Display-Technologie.

Da Antikörper-codierende Phagen-Display-Bibliotheken häufig eine Vielzahl an verschiedenen spezifischen Antikörpern auf ihrer Oberfläche präsentieren, sollte vor der Inkubation mit dem Testantigen sichergestellt werden, dass das Phagen-Display spezifische neutralisierende Antikörper, d. h. erste Moleküle, gegen das Zielantigen aufweist. Dafür ist im ersten Schritt optional eine Affinitätsselektion durch Adsorption von spezifischen Phagen an das Zielantigen nötig, und die neutralisierenden Eigenschaften sind mittels Neutralisationstest nachzuweisen. Anschließend kann diese spezifische Phagen-Präparation als Antikörper im Sinne der Erfindung genutzt werden. Sofern bereits eine spezielle Phagen Bibliothek für das Zielantigen vorliegt, kann der Schritt der Affinitätsselektion entfallen. Die eigentliche Adsorption an das Testantigen erfolgt mit der o.g. spezifischen Phagen-Präparation, die mit dem Testantigen in Verbindung gebracht wird, um die Absorption von neutralisierenden, Epitop-spezifischen Antikörpern zu ermöglichen. Diese vom Testantigen adsorbierten Antikörper werden in einem Erreger-spezifischen Neutralisationstest eingesetzt. Dadurch können Rückschlüsse auf die Immunogenität des Testantigens getroffen werden. Weisen die Antikörper neutralisierende Eigenschaften auf, so wird das Testantigen als immunogen angesehen. Je höher der Neutralisationstiter, desto besser ist die immunogene Fähigkeit des Testantigens.

Das zweite erfindungsgemäße Verfahren bildet *in vitro* die Bestimmung der Immunogenität des Testantigens im Vergleich zum Zielantigen nach. Die Kombination der Adsorption von Antikörpern an ein zu evaluierendes Testantigen als vorgeschalteten Schritt vor der Durchführung eines Neutralisationstests ermöglicht erstmals die Beurteilung der immunologischen Fähigkeiten eines Testantigens in-vitro, sodass kostspielige und langwierige Tierversuche auf ein Mindestmaß reduziert werden können. Das erfindungsgemäße Verfahren erlaubt mithin die Beurteilung von Epitopen ex-vivo, insbesondere deren Fähigkeit, neutralisierende Antikörper zu induzieren.

### Merkmale des ersten und des zweiten erfindungsgemäßen Verfahrens

Im Folgenden werden Einzelheiten des erfindungsgemäßen Verfahrens beschrieben, die sowohl für das erste erfindungsgemäße Verfahren als auch für das zweite erfindungsgemäße Verfahren gelten.

Es kann vorgesehen sein, dass das Testantigen in Schritt (a) an eine Oberfläche immobilisiert ist.

Es kann vorgesehen sein, dass Schritt (a) in einer Mikrotiterplatte, deren Oberfläche eine adsorptive Oberfläche ist, einem Reaktionsgefäß, dessen Oberfläche eine adsorptive Oberfläche ist, einem Säulensystem oder einem System, das Kügelchen mit adsorptiver Oberfläche aufweist, oder auf einer für Phagen-Displays spezifischen Oberfläche durchgeführt wird. Unter dem Begriff "adsorptive Oberfläche" wird eine Oberfläche mit adsorptiven Eigenschaften verstanden die dazu in der Lage ist, das Testantigen zu immobilisieren. In dem erfindungsgemäßen Verfahren kann vorgesehen sein, dass der Phage an sein Testantigen und/oder das Zielantigen bindet und anschließend selektioniert wird. Die Selektion kann mittels eines beliebigen Verfahrens durchgeführt werden, beispielsweise durch Dissoziation von der adsorptiven Oberfläche, Zentrifugation, FACS basiertes Sortieren oder ein anderes bekanntes Verfahren.

Die Inkubationsverhältnisse für die Adsorption in Schritt (a) werden vorzugsweise auf das verwendete Testantigen abgestimmt. So werden hitzeempfindliche Testantigene bevorzugt bei 4 °C inkubiert. Allgemein kann die Adsorptionstemperatur in Abhängigkeit der Stabilität der Testantigene sowie der Adsorptionszeit zwischen 4 °C und 56 °C variieren. Die Adsorptionszeit kann zwischen 2 h und 24 h, bevorzugt 16 h, betragen, um eine ausreichende Antikörperbindung an das Testantigen zu gewährleisten.

Lichtempfindliche Testantigene werden vorzugsweise in lichtdichten Reaktionsgefäßen bearbeitet.

Für die Adsorption sind verschiedene Systeme verwendbar. Neben der bereits beschriebenen Immobilisation des Testantigens auf einer 96er-Multikavitäten-Platte mit adsorptiven Eigenschaften oder dem belassen des Testantigens in flüssiger Phase können auch andere Reaktionsgefäße mit adsorptiven Eigenschaften, Säulensysteme oder Kügelchen-basierte Systeme (die auch als Beads-basierte Systeme bezeichnet werden) zum Einsatz kommen. Säulen- und Beads-basierte Systeme eignen sich insbesondere für mit speziellen Proteinen markierte Testantigene, sogenannte "getagte" Testantigene.

Die benötigte Menge an Testantigen ist abhängig vom verwendeten ersten Molekül, beispielsweise einem Antikörper, und dessen Konzentration.

Es kann vorgesehen sein, dass Schritt (b) das In-Kontakt-Bringen des oder der in Schritt (a) selektierten ersten Moleküle mit einem Krankheitserreger oder einer pathogenen Substanz umfasst. Bei dem Krankheitserreger kann es sich beispielsweise um ein Virus oder Bakterium handeln. Bei dem in Schritt (b) vorgesehenen Neutralisationstest kann es sich um einen Virus-Neutralisationstest handeln, wenn das oder die ersten Moleküle jeweils ein Antikörper sind.

Unter dem Begriff "Testantigen" wird die zu testende Struktur verstanden, an die Moleküle, beispielsweise Antikörper, binden können. Dabei kann es sich um Proteine, Peptide, Kohlenhydrate, Lipide, lebende oder abgetötete Erreger und um diesen Strukturen ähnliche Substanzen handeln. Testantigene werden vom Immunsystem eines Organismus als fremd erkannt und lösen dadurch die Bildung von Antikörpern aus. Diese Antikörper sind Epitop-spezifisch. Sie können neutralisierend oder nicht-neutralisierend sein.

Unter dem Begriff "Zielantigen" wird eine biologische Struktur verstanden, gegen welche später die schützende antikörpervermittelte Immunantwort gerichtet sein soll. In der Regel handelt es sich dabei um krankheitsauslösende Organismen oder Bestandteile krankheitsauslösender Organismen. Oberflächenproteine von Zellen sind Zielantigene im Sinne der vorliegenden Erfindung.

Unter dem Begriff "Antikörper" werden Immunglobuline und Immunglobulin enthaltende Formulierungen verstanden. Die verwendeten Antikörper müssen neutralisierende, Epitop-spezifische Eigenschaften gegen das "Zielantigen" aufweisen. Bei den Antikörpern kann es sich um Seren, monoklonale Antikörper, Serumpräparationen, synthetisch hergestellte Antikörper, Hybridomaüberstände und andere Antikörper enthaltende Ex- und Sekrete handeln. Ein Antikörper mit Epitop-spezifischen, neutralisierenden Eigenschaften bindet an die Epitope des Zielantigens, welche für die Infektiosität eines krankheitsauslösenden Erregers essentiell sind. Dadurch kann eine Infektion und/oder die Vermehrung des Erregers gehemmt werden.

Unter dem Begriff "Immunogenität eines Antigens" wird die Fähigkeit eines Antigens verstanden, bei Applikation in einen Organismus eine schützende Immunantwort auslösen zu können, welches sich z. B. durch die Bildung spezifisch neutralisierender Antikörper zeigt.

Das erfindungsgemäße Verfahren ermöglicht unter anderem folgende spezielle Verfahrensausgestaltungen. Dazu gehören die Testung von Antigenen, d. h. den Testantigenen, auf das Vorhandensein von nicht linearen neutralisierenden Epitopen; der Ersatz der *In*-*vivo*-Testung von Antigenen, d. h. den Testantigenen, auf deren Immunogenität in Tierversuchen durch ein *In*-*vitro*-Verfahren im Labor; die Untersuchung von antigenetischen Kreuzreaktionen verschiedener Antigene, d. h. verschiedener Testantigene; und die Adsorption von unerwünschten Antikörpern aus einem biologischen Material (z. B. Blut oder Gelenkflüssigkeit) als eine Art "Blutwäsche".

Nach Maßgabe der Erfindung ist ferner ein Kit zur In-vitro-Bestimmung der Immunogenität eines ersten Antigens, das als Testantigen bezeichnet wird, vorgesehen. Das Kit enthält
(i) ein Molekülgemisch, das zumindest ein erstes Molekül und zumindest ein zweites Molekül enthält, zur Adsorption an dem Testantigen; und
(ii) ein zweites Antigen, das als Zielantigen bezeichnet wird, zur Durchführung eines Neutralisationstests unter Verwendung des zumindest einen ersten Moleküls.
Es kann vorgesehen sein, dass das erfindungsgemäße Kit ferner einen Testkörper mit einer Oberfläche zur Immobilisierung des Testantigens aufweist. In einer Ausführungsform enthält das erfindungsgemäße Kit ferner Mittel zur Durchführung eines Neutralisationstests.

Nach Maßgabe der Erfindung ist ferner die Verwendung des erfindungsgemä-ßen Kits zur In-vitro-Bestimmung der Immunogenität eines Antigens vorgesehen. Das erfindungsgemäße Kit ist insbesondere zur Durchführung des erfindungsgemäßen Verfahrens geeignet.

Einzelheiten des erfindungsgemäßen Kits und der erfindungsgemäßen Verwendung sind bereits im Zusammenhang mit dem erfindungsgemäßen Verfahren beschrieben worden. Auf diese Einzelheiten wird verwiesen.

Die Erfindung wird nachstehend anhand von Ausführungsbeispielen, die die Erfindung nicht einschränken sollen, unter Bezugnahme auf die Zeichnungen näher erläutert. Dabei zeigen
- Fig. 1: ein Ablaufschema einer Ausführungsform des ersten erfindungsgemäßen Verfahrens; und
- Fig. 2: ein Ablaufschema einer Ausführungsform des zweiten erfindungsgemäßen Verfahrens.

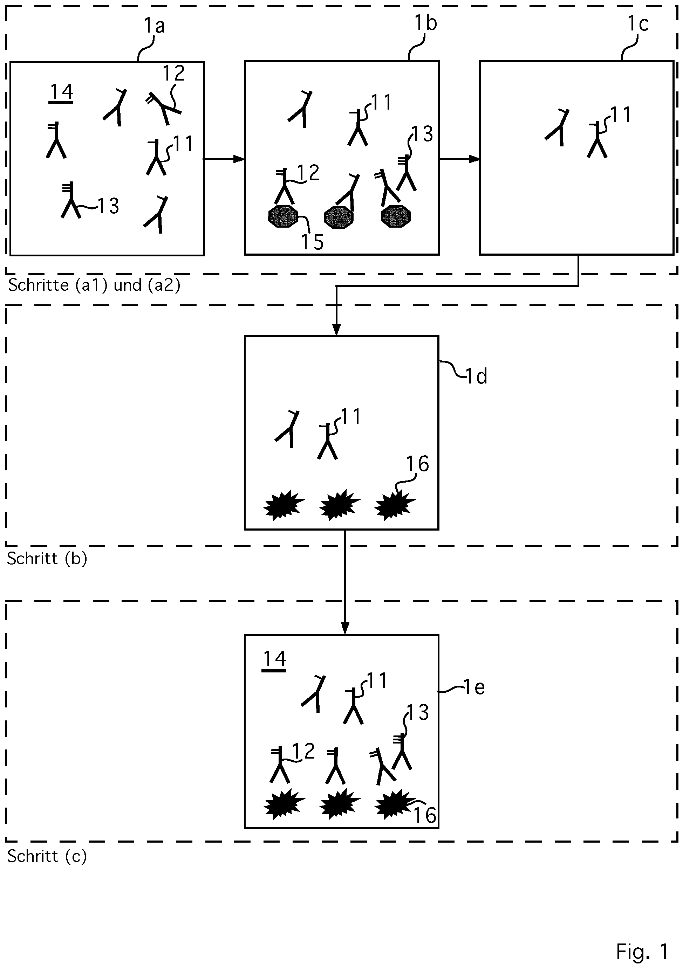

Das in Fig. 1 gezeigte Ablaufschema einer Ausführungsform des ersten erfindungsgemäßen Verfahrens basiert auf der negativen Selektion von Antikörpern 11. Die Antikörper 11 sind Epitop-spezifische, neutralisierende Antikörper und im Sinne der Erfindung erste Moleküle. Die Antikörper 11 sind gemeinsam mit Antikörpern 12 und 13 in einem antikörperspezifischen Serum 14 enthalten. Die Antikörper 12, 13 sind zweite und dritte Moleküle. Zumindest ein Antikörper 12 adsorbiert an das Testantigen, besitzt aber keine gegen das zu untersuchende Epitop des Testantigens neutralisierende Wirkung. Kasten (1a) veranschaulicht das Serum 14.

Das Serum 14 wird zur Selektion von Antikörpern 11 in Kontakt mit einem Testantigen 15 gebracht. Je nach immunologischer Fähigkeit des Testantigens, neutralisierende Antikörper zu induzieren, binden Antikörper 11 an das Testantigen 15 (Kasten (1b)). Es ist in Kasten (1b) zu erkennen, dass zumindest ein Antikörper 12 an dem Testantigen adsorbiert wird. Weiterhin können Antikörper 13 an das Testantigen binden. Diejenigen Antikörper 11, die nicht vom Testantigen adsorbiert werden, werden im nächsten Schritt abgenommen (Kasten (1c)). Die Kästen (1a), (1b) und (1c) veranschaulichen somit die Schritte (a1) und (a2) des ersten erfindungsgemäßen Verfahrens, d. h. die negative Selektion des ersten Moleküls.

Die abgenommenen Antikörper 11 werden zum Durchführen eines Neutralisationstests unter Verwendung des Zielantigens 16 verwendet (Kasten (1d)). Auf diese Weise wird ein Neutralisationstiter erhalten. Kasten (1d) veranschaulicht somit Schritt (b) des erfindungsgemäßen Verfahrens. Bei dem in Kasten (1d) gezeigten Zielantigen 16 kann es sich um ein Epitop auf einem Virus handeln.

Anschließend ist ein Vergleich des Neutralisationstiters mit einer Positivkontrolle vorgesehen (Kasten (1e)). Die Positivkontrolle sieht die Bestimmung eines weiteren Neutralisationstiters vor, der im Folgenden als Vergleichstiter bezeichnet wird. Kasten (1e) veranschaulicht die Ermittlung eines Vergleichstiters unter Verwendung des Zielantigens 16 und des unbehandelten Serums 14, d. h. in Gegenwart aller Antikörper, die in dem Serum 14 enthalten sind, also den Antikörpern 11, 12 und 13.

Durch den Vergleich des in Schritt (b) bestimmten Neutralisationstiters mit dem Vergleichstiter kann die Immunogenität des Testantigens bestimmt werden. Das entspricht Schritt (c) des erfindungsgemäßen Verfahrens. Ist der in Schritt (b) bestimmte Neutralisationstiter geringer als der Vergleichstiter, trägt das Testantigen Epitope, die neutralisierende Antikörper binden können. Damit ist es potenziell dazu in der Lage, die Bildung neutralisierende Antikörper zu induzieren. Werden verschiedene Testantigene mit demselben Antikörper getestet, besitzt das Testantigen mit dem geringsten in Schritt (b) bestimmten Neutralisationstiter die meisten Epitope, die neutralisierende Antikörper binden können. Grundvoraussetzung ist dann, dass identische Mengen Antikörper und Testantigen eingesetzt werden.

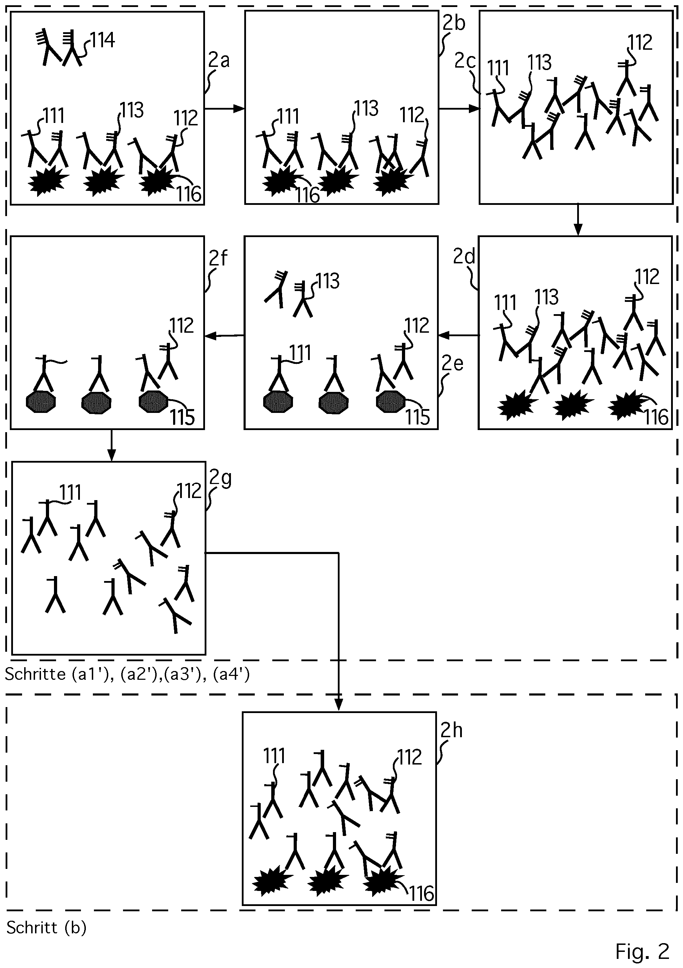

Das in Fig. 2 gezeigte Ablaufschema einer Ausführungsform des zweiten erfindungsgemäßen Verfahrens basiert auf der positiven Selektion eines Antikörpers 111. Antikörper 111 ist ein erstes Molekül. Antikörper 111 sind gemeinsam mit Antikörpern 112, Antikörpern 113 und Antikörpern 114 in einem Molekülgemisch enthalten. Die Antikörper 111 sind erste Moleküle, die Antikörper 112, 113 und 114 zweite, dritte und vierte Moleküle.

Kasten (2a) veranschaulicht das In-Kontakt-Bringen des Molekülgemisches mit einem Zielantigen 116. Das Molekülgemisch wird mit dem Zielantigen 116 inkubiert, um die Antikörper zu selektieren, die an das Zielantigen 116 binden. Kasten (2a) zeigt, dass die Antikörper 111, 112 und 113 an dem Zielantigen 116 adsorbieren, Antikörper 114 aber nicht. Anschließend werden die ungebundenen Antikörper, also Antikörper 114, weggewaschen. Die Antikörper 111, 112 und 113 sind die selektierten Antikörper. Die Kästen (2a) und (2b) entsprechen Schritt (a1') des zweiten erfindungsgemäßen Verfahrens. Bei dem in Kasten (2a) gezeigten Zielantigen 116 kann es sich um ein Epitop auf einem Virus handeln.

Die Antikörper 111, 112 und 113 werden anschließend vermehrt (Kasten (2c)). Das entspricht Schritt (a2') des zweiten erfindungsgemäßen Verfahrens. Danach werden neutralisierende Eigenschaften der selektierten Antikörper, also der Antikörper 111, 112 und 113, untersucht (Kasten (2d)), indem ein Neutralisationstest mit dem Zielantigen 116 des Virus durchgeführt wird. Sobald ein Titer angezeigt wird, haben die selektierten Antikörper, hier die Antikörper 111, 112 und 113, neutralisierende Wirkung. Kasten (2d) entspricht Schritt (a3') des zweiten erfindungsgemäßen Verfahrens. Am Schluss dieses Verfahrensschrittes liegt ein gegen das Zielantigen gerichtetes Antikörpergemisch vor.

Kasten (2e) veranschaulicht die Selektion der Antikörper aus dem gewonnen Molekülgemisch, die an ein Testantigen 115 binden können. Das entspricht Schritt (a4') des zweiten erfindungsgemäßen Verfahrens. Die in Schritt (a3') vermehrten Antikörper 111, 112 und 113 werden mit dem Testantigen 115 inkubiert, um die Adsorption dieser Antikörper 111, 112 und 113 an dem Testantigen 115 zu ermöglichen. In Kasten (2e) ist gezeigt, dass die Antikörper 111 und 112 an dem Testantigen 115 adsorbieren, Antikörper 113 aber nicht. Das heißt, nur die Antikörper 111 und 112 besitzen eine gegen das Testantigen spezifische Wirkung. Anschließend werden nicht gebundene Antikörper, also Antikörper 113, weggewaschen (Kasten (2f)). Die an das Testantigen 115 gebundenen Antikörper, das sind die Antikörper 111 und 112, sind die selektierten Antikörper, die nun vermehrt werden (Kasten (2g)). Das entspricht Schritt (a5') des zweiten erfindungsgemäßen Verfahrens.

Die Antikörper 111, 112 werden zum Durchführen eines Neutralisationstests unter Verwendung des Zielantigens 116 verwendet (Kasten (2h)). Auf diese Weise wird ein Neutralisationstiter erhalten. Kasten (2h) veranschaulicht somit Schritt (b) des erfindungsgemäßen Verfahrens.

Anhand des in Schritt (b) erhaltenen Neutralisationstiters kann die Immunogenität des Testantigens bestimmt werden (Schritt (c) des erfindungsgemäßen Verfahrens). Im Gegensatz zum ersten erfindungsgemäßen Verfahren sind keine weiteren Schritte notwendig. Die bloße Anzeige eines Neutralisationstiters beweist die neutralisierende Wirkung der Antikörper, hier der Antikörper 111. Antikörper 112 sind zwar gegen das Testantigen spezifisch, haben aber keine neutralisierende Wirkung und wirken sich daher nicht auf den Neutralisationstiter aus.

In einer bevorzugten Ausführungsform wird mit dem erfindungsgemäßen Verfahren nicht nur ein Testantigen, sondern mindestens 2 untersucht. In diesem Fall ist der Vergleich der Neutralisationstiter verschiedener Testantigene unter dem Einsatz der gleichen Antikörperkonzentrationen bevorzugt.

### Beispiele 1 bis 3

Die Beispiele 1 und 2 sind Beispiele für das erste erfindungsgemäße Verfahren. Das erste erfindungsgemäße Verfahren sieht eine negative Selektion vor. Die einzelnen Bearbeitungsschritte sind an sich bereits aus dem Stand der Technik bekannt. Das erste erfindungsgemäße Verfahren ermöglicht jedoch durch die vorgeschaltete Adsorption von Antikörpern an das zu untersuchende Testantigen im Gegensatz zum Stand der Technik *in vitro* die Bestimmung der Immunogenität eines Testantigens im Vergleich zu einem Zielantigen im Neutralisationstest. Beispiel 3 beschreibt eine Ausführungsform eines Neutralisationstests.

### Beispiel 1

In diesem Beispiel wurde das Testantigen in einer 96er-Multiwellplatte mit adsorptiven Eigenschaften immobilisiert. Dazu wurde die Antigen-Präparation in die Kavitäten pipettiert und über Nacht bei 4 °C in einer feuchten Kammer gelagert. Danach erfolgte 3-maliges Waschen der Kavitäten mit 100 µl Phosphat-gepufferter Salzlösung (PBS). Das PBS wurde abgenommen und unspezifische Reaktionen wurden durch das Auftragen eines Blockierungs-Puffers geblockt. Nach Abnahme des Blockierungs-Puffers erfolgte das Auftragen des Antikörpers. Der Ansatz wurde 2 h bei 37 °C, 90 % Luftfeuchte inkubiert. Der Antikörper wurde nun abgenommen und in einem Zielantigen-Neutralisationstest eingesetzt.

### Beispiel 2

In diesem Beispiel wurde das Testantigen in Lösung belassen oder gebracht. Diese Anwendung setzt voraus, dass das Testantigen und die ihm beigemischten Substanzen den Serumneutralisationstest nicht beeinflussen. Das Testantigen in Lösung wurde mit dem Antikörper versetzt. Der Ansatz wurde 2 h bei 37 °C, 90 % Luftfeuchte inkubiert. Das Antikörper-Antigengemisch wurde nun abgenommen und in einem Zielantigen-Neutralisationstest eingesetzt.

### Beispiel 3: Serumneutralisationstest

Das Beispiel beschreibt die Durchführung eines Neutralisationstests. Dabei handelt es sich um einen Serumneutralisationstest, wie er bereits aus dem Stand der Technik bekannt ist.

Für den Serumneutralisationstest wurde der RVA-Virenstamm OSU mit Dulbecco's Modification of Eagle Medium (DMEM), ergänzt mit hoher Glukose, Natriumpyruvat und GlutaMAX^{™} (Thermo Fisher Scientific, Deutschland) mit 10 µg/ml Trypsin verdünnt und bei 37 °C, 5 % CO₂ und 90 % Luftfeuchtigkeit aktiviert. Serielle 2-fache Verdünnungen von zuvor der Adsorption zugeführten Antikörpern wurden in eine 96-Well-Platte mit einer Anfangsverdünnung von 1: 10 gegeben. Das RVA wurde mit einer Multiplicity of infection (m.o.i.) von 0,025 in den Assay eingesetzt. Nach dem Mischen von 25 µl der Antikörperverdünnung und 25 µl Virussuspension wurde die Mischung bei 37 °C, 5 % CO₂ und 90 % Luftfeuchtigkeit für 2 Stunden kultiviert. Danach wurde eine Suspension mit 0,4 x 10⁵ MA104 Zellen/50 l Dulbecco's Modified Eagle Medium (DMEM) in die Vertiefungen mit der Serum-Virus-Suspension eingesät. Der Serumneutralisationstest wurde bei 37 °C, 5 % CO₂ und 90 % Luftfeuchtigkeit für 24 h kultiviert. Danach wurden die Zellen einmal mit phosphatgepufferter Salzlösung (PBS) gewaschen, mit 80%igem Aceton bei -20 °C für 10 min fixiert und erneut zweimal mit PBS gewaschen.

Zur Auswertung wurde ein Immunfluoreszenz-Test durchgeführt. Als primärer Antikörper wurde ein gereinigtes Antikörperpräparat aus Kaninchenseren nach Impfung mit einem rekombinanten E.coli exprimierten VP6 von RVA verwendet. Der Serumneutralisationstest-Titer wurde als die höchste reziproke Serumverdünnung ausgedrückt, die eine 100%ige FFU-Reduktion ergibt.

In diesem Beispiel besitzt das OSU-spezifische Serum einen Serumneutralisationstest-Titer von 80. Durch die Adsorption von neutralisierenden Antikörpern an das Testantigen wird die verfügbare Menge an neutralisierenden Antikörpern, die gegen das Zielantigen gerichtet sind (RVA OSU) vermindert, sodass das OSU-spezifische Serum nun einen geringeren Serumneutralisationstiter von 20 aufweist. Testantigene, die keine neutralisierenden Antikörper adsorbieren, verringern dagegen den Serumneutralisationstiter des OSU-spezifischen Serums nicht.

### Bezugszeichenliste

- 11: Antikörper
- 12: Antikörper
- 13: Antikörper
- 14: Serum
- 15: Testantigen
- 16: Zielantigen

- 111: Antikörper
- 112: Antikörper
- 113: Antikörper
- 114: Antikörper
- 115: Testantigen
- 116: Zielantigen

## Patentansprüche

1. Verfahren zur In-vitro-Bestimmung der Immunogenität eines ersten Antigens, das als Testantigen bezeichnet wird, wobei das Verfahren umfasst:
(a) Selektieren zumindest eines ersten Moleküls durch Inkubieren des Testantigens mit einem Molekülgemisch, das das zumindest eine erste Molekül und zumindest ein zweites Molekül enthält, um die Adsorption des zumindest einen ersten Moleküls oder des zumindest einen zweiten Moleküls an dem Testantigen zu ermöglichen,
(b) Durchführen eines Neutralisationstests unter Verwendung eines zweiten Antigens, das als Zielantigen bezeichnet wird, und unter Verwendung des in Schritt (a) selektierten, zumindest einen ersten Moleküls unter Erhalt eines Neutralisationstiters; und
(c) Bestimmen der Immunogenität des Testantigens unter Verwendung des in Schritt (b) erhaltenen Neutralisationstiters.

2. Verfahren nach Anspruch 1, **dadurch gekennzeichnet, dass** Schritt (a) die Schritte umfasst:
(a1) Bereitstellen eines Serums, das das Molekülgemisch enthält; und
(a2) Inkubieren des in Schritt (a) bereitgestellten Serums mit dem Testantigen, um die Adsorption des zumindest einen zweiten Moleküls an dem Testantigen zu ermöglichen, zur Selektion des zumindest einen ersten Moleküls, das nicht an das Testantigen bindet.

3. Verfahren nach Anspruch 1 oder Anspruch 2, **dadurch gekennzeichnet, dass** Schritt (c) den Vergleich des in Schritt (b) bestimmten Neutralisationstiters mit einem Vergleichs-Neutralisationstiter umfasst, wobei der Vergleichsneutralisationstiter durch Durchführen eines Neutralisationstests unter Verwendung der in dem Serum enthaltenen Moleküle und unter Verwendung des Zielantigens bestimmt wird.

4. Verfahren nach Anspruch 1, **dadurch gekennzeichnet, dass** Schritt (a) zumindest die Teilschritte umfasst:
(a1') Selektieren des zumindest einen ersten Moleküls, das an das Zielantigen bindet, durch Inkubieren des Molekülgemisches mit dem Zielantigen, um die Adsorption des zumindest einen ersten Moleküls an dem Zielantigen zu ermöglichen;
(a2') Vermehren des zumindest einen ersten Moleküls, das in Schritt (a1') an das Zielantigen binden konnte; und
(a3') Durchführen eines Neutralisationstests zur Bestimmung einer neutralisierenden Eigenschaft des in Schritt (a2') vermehrten, zumindest einen ersten Moleküls unter Erhalt eines Neutralisationstiters.

5. Verfahren nach Anspruch 4, **dadurch gekennzeichnet, dass** es im Anschluss an Schritt (a3') die Teilschritte umfasst:
(a4') Inkubieren des in Schritt (a1') selektierten, zumindest einen ersten Moleküls, wenn in Schritt (a3') ein Neutralisationstiter erhalten wird, mit dem Testantigen; und
(a5') Vermehren des zumindest einen ersten Moleküls, das in Schritt (a4') an das Testantigen binden konnte.

6. Verfahren nach einem der vorstehenden Ansprüche, **dadurch gekennzeichnet, dass** das zumindest eine erste Molekül und/oder das zumindest eine zweite Molekül jeweils Antikörper sind.

7. Verfahren nach einem der vorstehenden Ansprüche, **dadurch gekennzeichnet, dass** das Testantigen aus der Gruppe ausgewählt ist, die aus einem Protein, einem Peptid, einem Kohlenhydrat, einem Lipid, einem lebenden Erreger und einem abgetöteten Erreger besteht.

8. Verfahren nach einem der vorstehenden Ansprüche, **dadurch gekennzeichnet, dass** in Schritt (a) das Testantigen an eine Oberfläche immobilisiert ist.

9. Verfahren nach Anspruch 8, **dadurch gekennzeichnet, dass** Schritt (a) in einer Mikrotiterplatte, deren Oberfläche eine adsorptive Oberfläche ist, einem Reaktionsgefäß, dessen Oberfläche eine adsorptive Oberfläche ist, einem Säulensystem oder einem System, das Kügelchen mit adsorptiver Oberfläche aufweist, auf einer für Phagen-Displays spezifischen, absorptiven Oberfläche, durchgeführt wird.

10. Verfahren nach einem der vorstehenden Ansprüche, **dadurch gekennzeichnet, dass** Schritt (b) das In-Kontakt-Bringen des selektierten, zumindest einen ersten Moleküls mit einem Krankheitserreger oder einer pathogenen Substanz umfasst.

11. Verfahren nach einem der vorstehenden Ansprüche, **dadurch gekennzeichnet, dass** der Krankheitserreger ein Virus oder Bakterium ist.

12. Kit zur In-vitro-Bestimmung der Immunogenität eines ersten Antigens, das als Testantigen bezeichnet wird, enthaltend
(i) ein Molekülgemisch, das zumindest ein erstes Molekül und zumindest ein zweites Molekül enthält, zur Adsorption an dem Testantigen; und
(ii) ein zweites Antigen, das als Zielantigen bezeichnet wird, zur Durchführung eines Neutralisationstests unter Verwendung des zumindest einen ersten Moleküls.

13. Kit nach Anspruch 12, **dadurch gekennzeichnet, dass** es ferner einen Testkörper mit einer Oberfläche zur Immobilisierung des Testantigens aufweist.

14. Kit nach Anspruch 12 oder Anspruch 13, **dadurch gekennzeichnet, dass** es ferner Mittel zur Durchführung eines Neutralisationstests enthält.

15. Verwendung eines Kits nach einem der Ansprüche 12 bis 14, zur In-vitro-Bestimmung der Immunogenität eines Antigens.
